# EUROPÄISCHE PATENTSCHRIFT

(11) **EP 0 724 575 B1**
(45) Veröffentlichungstag und Bekanntmachung des Hinweises auf die Patenterteilung: **17.09.1997**
(21) Anmeldenummer: 94928883.1
(22) Anmeldetag: 10.10.1994
(51) Int. Cl.: C07D 251/16

(54) **VERFAHREN ZUR HERSTELLUNG UNSYMMETRISCH SUBSTITUIERTER TRIAZINE**
METHOD OF PREPARING UNSYMMETRICALLY SUBSTITUED TRIAZINES
PROCEDE DE PREPARATION DE TRIAZINES SUBSTITUEES ASYMETRIQUEMENT

(30) Priorität: 19.10.1993 DE 4335497
(43) Veröffentlichungstag der Anmeldung: 07.08.1996
(73) Patentinhaber: BASF AKTIENGESELLSCHAFT, 67056 Ludwigshafen (DE)
(72) Erfinder: SCHÄFER, Bernd, D-76889 Dierbach (DE); MAYER, Horst, D-67069 Ludwigshafen (DE)
(86) Internationale Anmeldenummer: EP9403331
(87) Internationale Veröffentlichungsnummer: WO9511237

(56) Entgegenhaltungen:
- US-A- 4 886 881

## Beschreibung

Die vorliegende Erfindung betrifft ein Verfahren zur Herstellung unsymmetrisch substituierter Triazine der allgemeinen Formel I 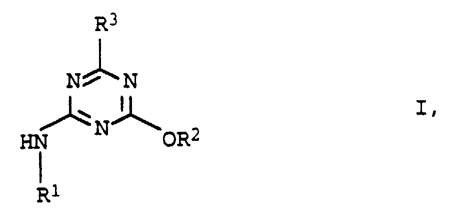 in der R¹ Wasserstoff, Methyl oder Ethyl, R² und R³ unabhängig voneinander einen Kohlenwasserstoffrest mit 1 bis 6 C-Atomen, der unter den Reaktionsbedingungen inerte Substituenten tragen kann, bedeuten, durch Umsetzung von Cyanguanidin der Formel II 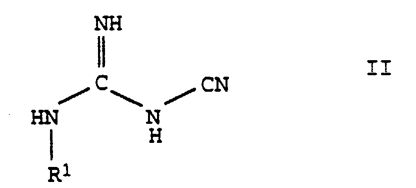 mit einem Carbonsäurederivat in Gegenwart eines Alkohols der Formel III

R²-OH III.

Unsymmetrisch substituierte Triazine lassen sich auf sehr vielfältige Weise herstellen, z.B. ausgehend von N-Cyanamiden durch Umsetzung mit Vilsmeier-Komplexen (R.L.N. Harries, Aust. J. Chem. 34 (1981) 623), aus N-Cyanimidosaureester (DE-A 34 11 202; M.A. Pérez, J.L. Soto, Heterocycles, 20 (1983) 463; K.R. Huffman, F.C. Schaefer, J. Org. Chem. 28 (1963) 1816) oder aus Biguanidinen (S.L. Shapiro et al, J. Org. Chem. 25 (1960) 379; US-A 2 535 968). Publiziert ist auch die Umsetzung von Guanylthioharnstoff mit Dimethylsulfat und Carbonsäurederivaten (H. Eilingsfeld, H. Scheuermann, Chem. Ber. 100 (1967) 1874; DE-A 16 70 147; EP-A 545 149) sowie die Umsetzung von Trichloracetamidinoguandinen mit Derivaten der Trifluoressigsäure (DE-A 40 34 078). Nach all diesen Methoden werden keine Chelatkomplexe als Zwischenprodukte durchlaufen.

Eine andere Möglichkeit zur Herstellung von 6-Trifluormethyl-1,3,5-triazinen besteht nach einem aus Yakugaku Zasshi 95, 499-511 (1975) bekannten Verfahren darin, N-Cyanoguanidine in Kupferkomplexe von N-Amidino-O-alkylisoharnstoffen zu überführen, die Harnstoffderivate mit Schwefelwasserstoff freizusetzen und anschließend mit Trifluoressigsäureester gemäß folgendem Reaktionsschema umzusetzen:

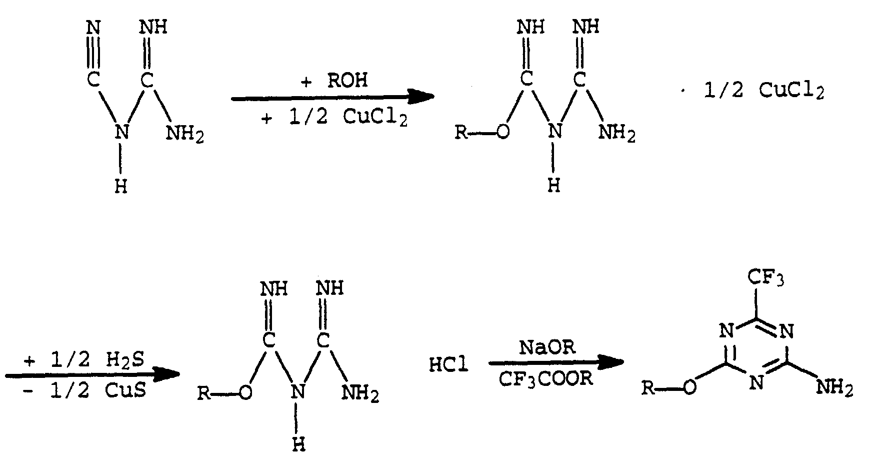

Aus der DD-A-252 374 ist eine Variante dieses Verfahrens bekannt, wobei anstelle des Kupfer(II)-chlorides das Acetat des Kupfers verwendet wird.

Für diese Verfahren sind stöchiometrische Mengen von Cu-Salzen nötig; in Abwesenheit von Cu-Salzen wird statt des N-Amidino-O-alkylisoharnstoffs hauptsächlich Guanylharnstoff gebildet (Kyushu Kogyo Daigaku Kenkyu Hokoku No. 12, 69-78 (1962).

Die Umsetzung von N-Amidino-O-alkylisoharnstoffhydrochlorid mit Ethylchloracetat in Ethanol/NaOC₂H₅ führt nur in schlechten Ausbeuten zum entsprechenden Chlormethyltriazin. Das Ausgangsprodukt mußte zuvor aus dem Cu-Komplex freigesetzt werden.

Die Herstellung von Chelatkomplexen ausgehend von Cyanguanidin mit Kupferacetat oder Zinkchlorid in Methanol wird von R.I. Dutta und A. Syamal in Coord. Chem. Rev., Bd. 2, 1967, S. 441-457 beschrieben. Aus Chemistry of Heterocyclic Compounds, Bd. 25, 1989, S. 547-550 ist bekannt, solche Zinkkomplexe zu isolieren und dann mit Trifluoressigsäureanhydrid zum Triazin umzusetzen.

In ähnlicher Weise bildet Zinksulfat einen Chelatkomplex, der nach Aufarbeitung durch Kochen in Wasser in einem zweiten Reaktionsschritt zu dem Sulfat des Amidino-O-methylisoharnstoffs zersetzt (US-A 3,360,534, IN-A 167 500) und dieser in einem dritten Reaktionsschritt mit Essigsäureanhydrid (S. Lotz, G. Kiel, G. Gattow, Z. anorg. allg. Chem. 604 (1991) 53-62) oder mit Trifluoressigsäuremethylester (T. Tsujikawa, Yakugaku Zasshi 95, loc. cit) zum Triazin in Ausbeuten von 31 bzw. 26 % umgesetzt werden kann.

Bei den zuletzt aufgeführten Verfahren handelt es sich stets um sehr aufwendige mehrstufige Verfahren, die im Fall der Ester nur in schlechten Ausbeuten zu den gewünschten Triazinen führen oder im Fall der Anhydride zwangsweise die Produktion stöchiometrischer Mengen Carbonsäure beinhalten, die nur sehr aufwendig in die entsprechenden Anhydride recycliert werden können. In aller Regel müssen die toxikologisch nicht unbedenklichen, gelegentlich schwer filtrierbaren Schwermetallkomplexe isoliert und dann in einem inerten Lösungsmittel mit den Anhydriden umgesetzt werden, da sich eine Umsetzung in alkoholischer Lösung aufgrund der Reaktion von Anhydriden mit Alkoholen verbietet. Des weiteren sind die beschriebenen Verfahren insofern nachteilig, als unabdingbar große Mengen an Schwermetallsalzen in Form organischer Schlämme anfallen, die nur schwer entsorgt werden können.

US-A 4,886,881 lehrt die einstufige Synthese von 2-Aminotriazinen, ausgehend von Cyanguanidin und Trimethylorthoacetat in Gegenwart eines Lewissäurekatalysators wie Zinkchlorid. Als Lösungsmittel werden Dimethylformamid und Acetonitril empfohlen.

Der Erfindung lag nun die Aufgabe zugrunde, ein Verfahren zu finden, das Triazine der Struktur I durch Umsetzung von Cyanguanidinen mit Carbonsäureestern, die leichter verfügbar aber auch weniger reaktiv sind, als entsprechende Anhydride oder Orthoester, zugänglich macht. Dieses Verfahren sollte nach Möglichkeit ohne Isolierung von Zwischenstufen (Eintopfverfahren) durchführbar sein.

Demgemäß wurde ein Verfahren zur Herstellung unsymmetrisch substituierter Triazine der allgemeinen Formel I 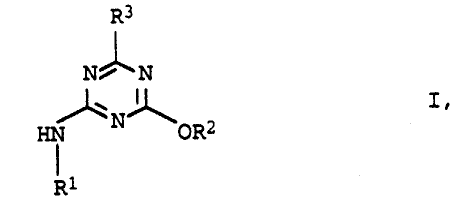 in der R¹ Wasserstoff, Methyl oder Ethyl, R² und R³ unabhängig voneinander einen Kohlenwasserstoffrest mit 1 bis 6 C-Atomen, der unter den Reaktionsbedingungen inerte Substituenten tragen kann, bedeuten, durch Umsetzung von Cyanguanidinen der Formel II 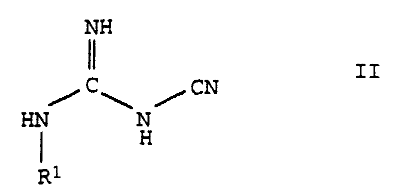 mit einem Carbonsäurederivat in Gegenwart eines Alkohols der Formel III

R²-OH III,

gefunden, das dadurch gekennzeichnet ist, daß man einen Carbonsäureester der Formel IV

R³-COOR⁴ IV,

in der R³ die obengenannte Bedeutung hat und R⁴ einen Kohlenwasserstoffrest mit 1 bis 6 C-Atomen darstellt, der unter den Reaktionsbedingungen inerte Substituenten tragen kann, in Gegenwart einer Base oder eines Carbonsäureamids, ausgewählt aus der Gruppe N,N-Di(C₁-C₄-alkyl) formamid, N,N-Di(C₁-C₄-alkyl)acetamid oder N-Methylpyrrolidon und in Gegenwart eines Salzes oder einer salzartigen Verbindung der Elemente Magnesium, Calcium, Aluminium, Zink, Kupfer, Eisen, Cobalt, Nickel oder Chrom umsetzt.

Das als Ausgangsstoff dienende substituierte Cyanguanidin ist allgemein bekannt. Die N-Methyl bzw. N-Ethyl substituierten Derivate sind aus Cyanguanidin und Dialkylsulfat wie von A.E. Kretov und A.S. Bespalyi in Zhur. Prik. Khimii, Bd. 34, (1961) 621ff. beschrieben, zugänglich. Das Guanidin kann auch in Form eines Säureadditionssalzes eingesetzt werden, wobei in diesem Fall die während der Reaktion frei werdende Säure zweckmäßig durch Zugabe einer geeigneten Base wie Natriummethanolat neutralisiert wird.

Als Kohlenwasserstoffrest im Triazin I sind für R² insbesondere aliphatische, cycloaliphatische, aromatische oder araliphatische Reste mit bis zu 8 Kohlenstoffatomen wie C₁-C₈-Alkyl, C₃-C₈-Alkenyl, C₃-C₈-Alkinyl, C₃-C₈-Cycloalkyl sowie Phenyl, Benzyl oder Phenethyl zu nennen. Im Hinblick auf die Verwendung der herzustellenden Zwischenprodukte I steht R² beispielsweise für C₁-C₄-Alkyl wie Methyl, Ethyl, n-Propyl, Isopropyl, n-Butyl, sec.-Butyl oder tert.-Butyl; C₃-C₄-Alkenyl wie Prop-2-en-1-yl, 1-Methylprop-2-en-1-yl, But-2-en-1-yl oder But-3-en-1-yl; C₃-C₄-Alkinyl wie Prop-2-in-1-yl oder But-2-in-1-yl; C₃-C₆-Cycloalkyl wie Cyclopropyl, Cyclobutyl, Cyclopentyl oder Cyclohexyl; besonders bevorzugt C₁-C₄-Alkyl wie Methyl.

Der Rest R² kann seinerseits noch weitere unter den Reaktionsbedingungen inerte Substituenten wie z.B. Fluor oder Chlor, Phenyl, C₁-C₄-Alkyl, C₁-C₄-Halogenalkyl oder C₁-C₄-Alkoxy tragen.

Der Carbonsäureesterrest R⁴ hat die voranstehend für R² genannte Bedeutung und ist vorzugsweise mit R² identisch.

Der Rest R³ ist nach bisherigen Beobachtungen breit variierbar. Er kann z.B. die für R² im einzelnen aufgeführten Bedeutungen haben. Im Hinblick auf die bestimmungsgemäße Verwendung der Triazine I steht R³ bevorzugt für C₁-C₄-Halogenalkyl, insbesondere Fluor- oder Chlormethyl oder -ethyl wie CCl₃, CF₃, CF₂CF₃, CF₂Cl, CFCl₂, CH₂Cl, CHCl₂, CH₂F und CHF₂. Besonders bevorzugt sind perhalogenierte Alkylreste wie CCl₃, CF₃ und C₂F₅.

Bevorzugt werden C₁-C₆-Alkylester einer perfluorierten oder perchlorierten C₁-C₃-Carbonsäure als Carbonsäureester der Formel IV verwendet.

Erfindungsgemäß wird die Umsetzung des Cyanguanidins II mit dem Alkohol III (bzw. R⁴OH, entstanden durch Hydrolyse des Esters IV) in Gegenwart einer Base vorgenommen. Überraschenderweise läßt sich die Reaktivität der Metallchelatkomplexe dadurch so steigern, daß die Reaktion mit den Estern IV möglich ist, obgleich diese Ester weitaus weniger reaktiv sind als entsprechende Carbonsäureanhydride oder Orthoester.

Als Basen eignen sich anorganische und organische Basen. Als anorganische Basen werden Alkalimetall- und Erdalkalimetallhydroxide bevorzugt, und als organische Basen tertiäre Amine wie C₁-C₄-Trialkylamin, z.B. Triethylamin, Pyridin oder N-Methylmorpholin. Zweckmäßigerweise verwendet man als Base das Alkali- oder Erdalkalialkoholat des umzusetzenden Alkohols R²OH. Dieses Alkoholat kann auch in situ gebildet werden, z.B. aus dem entsprechenden Alkalimetall oder Natriumamid oder Natriumhydrid und dem Alkohol III.

Die Menge an Base beträgt üblicherweise 0,1 bis 2, insbesondere 0,8 bis 1,2 Moläquivalente, bezogen auf das Cyanguanidin II. Größere Mengen sind möglich, bringen in der Regel aber keine weiteren Vorteile.

Anstelle der Base oder zusätzlich kann auch ein Carbonsäureamid aus der Gruppe N,N-Di(C₁-C₄-alkyl)formamid, N,N-Di(C₁-C₄-alkyl)acetamid oder N-Methylpyrrolidon verwendet werden. Beispielsweise seien Dimethylformamid, Diethylformamid, Dimethylacetamid und Diethylacetamid genannt. Der Zusatz von Carbonsäureamiden hat sich insbesondere bei Einsatz von Schwermetallen, insbesondere Kupfer als Chelatbildner bewährt.

Die Menge an Carbonsäureamid liegt im allgemeinen bei 1 bis 30, insbesondere 5 bis 10 Moläquivalente, bezogen auf das Cyanguanidin II. Es kann auch vorteilhaft sein, das Carbonsäureamid als Lösungsmittel zu nutzen.

Als Salze oder salzartige Verbindungen der Erdalkalimetalle, des Aluminiums bzw. der Schwermetalle kommen im Reaktionsmedium gut lösliche Produkte in Betracht wie Halogenide, z.B. Fluoride, Chloride oder Bromide, Nitrate, Sulfate oder ggf. Phosphate, Alkoholate oder Acetate. Nach bisherigen Erkenntnissen kommt es, abgesehen von guter Löslichkeit auf die Art der Erdalkalimetall-bzw. Metallverbindung nicht an, so daß u.a. Kostengründe bei der Auswahl maßgebend sind. Beispielsweise seien folgende Verbindungen aufgeführt: MgCl₂, Mg(OCH₃)₂, CaCl₂, Zn(NO₃)₂, Cu(CH₃COO)₂, AlCl₃, AlBr₃, ZnCl₂, ZnBr₂, CuCl₂, NiBr₂, CrCl₃, CaO, Ca(NO₃)₂, Mg(NO₃)₂, MgO, ZnO, FeCl₂, FeCl₃, Fe(NO₃)₂, Fe(NO₃)₃. Besonders bevorzugt sind die Chloride, insbesondere CaCl₂ und ZnCl₂.

Ein Vorteil des erfindungsgemäßen Verfahrens liegt darin, daß man weitgehend oder vollständig auf die Anwesenheit von Schwermetallen verzichten und aus ökotoxikologischen Gründen auf weniger bedenkliche Elemente wie Magnesium- und insbesondere Calcium ausweichen kann. Demgemäß sind lösliche Salze dieser Elemente besonders bevorzugt. Sehr gute Ergebnisse werden aber auch mit Zinkverbindungen erzielt.

Die Salze bzw. salzartigen Verbindungen der o.g. Elemente können in stöchiometrischen Mengen oder vorzugsweise weniger als stöchiometrischen Mengen, bezogen auf das Cyanguanidin II eingesetzt werden, z.B. in Mengen von 0,001 bis 2 mol, insbesondere 0,005 bis 1,0 mol pro Mol II.

Bei Verwendung von Schwermetallen wird man deren Menge möglichst gering halten, und nur katalytische Mengen, z.B. unter 0,6 mol pro Mol Cyanguanidin einsetzen. Im Fall von Magnesium- und Calciumsalzen liegt die besonders bevorzugte Menge bei 0,01 bis 1,0, insbesondere 0,05 bis 0,5 mol. Natürlich können auch mehr als stöchiometrische Mengen, bezogen auf II, z.B. 1 bis 2 mol pro Mol II, eingesetzt werden, jedoch sprechen Wirtschaftlichkeitsgründe dafür, eher unterstöchiometrische Mengen einzusetzen.

Der Ausgangsstoff III kann zum einem in situ aus dem Carbonsäureester IV (R²=R⁴) gebildet werden oder wird den Reaktionsgemisch (bevorzugt als Lösungsmittel) zugesetzt. Bei Verwendung des Edukts III gleichzeitig als Lösungsmittel empfiehlt es sich, die Alkoholkomponente im Ester IV entsprechend zu wählen (d.h. R²=R⁴), um Nebenprodukte zu vermeiden.

Das Molverhältnis von Cyanguanidin II zum Alkohol III liegt im allgemeinen bei 1 zu 30, insbesondere 5 zu 10.

Der Carbonsäureester IV wird zweckmäßigerweise in einer Menge von 0,5 bis 10, insbesondere 1 bis 5 mol, pro Mol Cyanguanidin II verwendet. Als Ester IV sind besonders bevorzugt folgende Verbindungen zu nennen:

C₁-C₄-Alkan- oder Halogenalkancarbonsäureester wie Essigsäuremethylester, Essigsäureethylester, Propionsäuremethylester, Propionsäureethylester, Trifluoressigsäuremethylester, Difluoressigsauremethylester, Fluoressigsauremethylester, Trichloressigsauremethylester, Dichloressigsäuremethylester, Chloressigsäuremethylester, Trifluoressigsäureethylester, Pentafluorpropionsäure(m)ethylester, Pentachlorpropionsäure(m)ethylester. Im Hinblick auf die bestimmungsgemäße Verwendung der Triazine I als Zwischenprodukte für Pflanzenschutzmittel sind als Ausgangsstoffe IV Trifluoressigsauremethylester und Trifluoressigsäureethylester besonders bevorzugt.

Die Umsetzung von II mit III und IV kann in Substanz, d.h. ohne Zusatz von inerten Lösungsmitteln erfolgen. Vorteilhaft wird der Alkohol R²OH gleichzeitig als Lösungsmittel verwendet. Nach einer besonders bevorzugten Ausführungsform wird dann als Base das entsprechende Alkoholat gewählt.

Die Reaktionstemperatur liegt zwischen 0 und 200°C, insbesondere 20 bis 150°C, besonders bevorzugt bei der Rückflußtezperatur der Reaktionsmischung.

Besondere Bedingungen bezüglich des Drucks sind nicht erforderlich, im allgemeinen nimmt man die Umsetzung bei Atmosphärendruck oder unter dem Eigendruck des jeweiligen Reaktionsmediums vor.

Die Umsetzung kann kontinuierlich als auch diskontinuierlich durchgeführt werden. Bei der kontinuierlichen Arbeitsweise leitet man die Reaktionspartner vorzugsweise durch einen Rohrreaktor oder eine Rührreaktorkaskade.

Gemäß einer besonders bevorzugten Ausführungsform des erfindungsgemäßen Verfahrens erfolgt die Herstellung des Triazins I ohne Isolierung von Zwischenprodukten. Beispielsweise derart, daß man das Cyanguanidin II, den Ester IV und weniger als stöchiometrische Mengen des Erdalkalimetallsalzes oder der Metallsalze (bzw. salzartigen Verbindungen) im Alkohol R₂OH als Lösungsmittel vorlegt, die Base, z.B. das Alkalimetallalkoholat wie NaOR² oder KOR² zudosiert und das Gemisch auf Rückfluß erhitzt.

In der Regel bricht man die Reaktion in üblicher Weise ab, wenn kein Cyanguanidin im Reaktionsgemisch mehr nachgewiesen werden kann (z.B. mittels Dünnschichtchromatographie, Hochdruckflüssigkeitschromatographie oder Gaschromatographie).

Die Aufarbeitung auf das Verfahrensprodukt I hin erfolgt dann in der Regel nach herkömmlichen Verfahren wie Destillation, Filtration, Zentrifugation oder durch Zugabe von Wasser und anschließender Extraktion.

Die erhaltenen Rohprodukte können gewünschtenfalls weiter gereinigt werden, z.B. durch Kristallisation, Rektifikation oder mittels chromatographischer Methoden.

Die nach dem erfindungsgemäßen Verfahren auf einfache Weise herzustellenden Triazine I sind wertvolle Zwischenprodukte zur Synthese von Farbstoffen, Arzneimitteln und Pflanzenschutzmitteln, insbesondere Herbiziden, wie z.B. in den Druckschriften EP-A 508 348, EP-A 111 442 oder DE-A 40 38 430 beschrieben.

### Synthesebeispiele

### Beispiel 1

### 2-Amino-4-methoxy-6-trifluormethyl-1,3,5-triazin

21 g (0,25 mol) N-Cyanguanidin, 3,4 g (0,025 mol) wasserfreies Zinkchlorid und 160 g (1,25 mol) Trifluoressigsauremethylester werden in 400 ml Methanol vorgelegt, auf 50°C erwärmt und 50 g (0,27 mol) 30 %ige Natriummethylat-Lösung innerhalb von 10 Stunden zugepumpt. Anschließend wird das Lösungsmittel weitgehend entfernt, der Rückstand mit 250 ml Wasser und 250 ml verdünnter Salzsäure gewaschen und bei 60°C/20 mbar getrocknet. Es werden 43,2 g (0,22 mol, 89 %) 2-Amino-4-trifluormethyl-6-methoxy-1,3,5-triazin in Form eines farblosen Pulvers erhalten (HPLC: > 99 Gew.-%)
FP.: 161 - 163,5°C

### Beispiel 2

### 2-Amino-4-methoxy-6-trifluormethyl-1,3,5-triazin

56,6 g (0,5 mol) Calciumchlorid (98 %, gepulvert) und 210 g (2,5 mol) N-Cyanguanidin werden in 2 1 Methanol vorgelegt. Man erhitzt unter Rühren auf Rückflußtemperatur und rührt eine Stunde unter Rückfluß, worauf man eine homogene Lösung erhält. Danach kühlt man die Mischung auf Raumtemperatur ab und gibt 640 g (5,0 mol) Trifluoressigsäuremethylester und anschließend innerhalb von 25 Minuten 450 g (2,5 mol) einer Lösung von Natriummethanolat (30 Gew.-% in Methanol) hinzu, worauf sich ein weißer Niederschlag abscheidet. Nach 2 Stunden Erhitzen unter Rückfluß kühlt man das Gemisch auf Raumtemperatur ab und stellt durch Zugabe von konz. Salzsäure einen pH-Wert von etwa 6 ein. Danach destilliert man den Methanol ab, gibt nach und nach ca. 2 1 Wasser zu, trennt die abgeschiedenen, feinkristallinen, weißen Kristalle ab und trocknet sie im Vakuum.
Ausbeute: 402,4 g (2,07 mol; 83 % d.Th.).
¹H-NMR-Spektrum (270 MHz, CDCl₃, int. TMS, δ (ppm)): 6,45 br (1H); 5,88 br (1H); 4,03 s (3H).

### Beispiel 3

### 2-Amino-4-methoxy-6-trifluormethyl-1,3,5-triazin

84 g (1 mol) Cyanguanidin und 100 g (0,5 mol) Kupferacetat werden in 600 ml Methanol vorgelegt und 7 h auf Rückfluß erhitzt. Nach dem Abkühlen auf 20°C wird der Ansatz abgesaugt und der feste Kupferkonplex abgetrennt und im Vakuum getrocknet. 41,4 g (0,1 mol) dieses Rückstands werden in 200 ml Methanol vorgelegt und innerhalb von 15 bis 45 g (0,25 mol) 30 %ige Natriummethylat-Lösung zugetropft. Anschließend tropft man 76,8 g (0,6 mol) Trifluoressigsauremethylester zu und erhitzt 2 h auf Rückfluß. Man läßt auf 40°C abkühlen, filtriert einen rotvioletten Feststoff (28,6 g) ab und engt die Mutterlauge ein. Der Rückstand wird mit Wasser gewaschen und getrocknet. Es werden 16,4 g (84,5 mmol, 38 %) des o.g. Triazins erhalten.

### Beispiel 4

### 2-Amino-4-methoxy-6-trifluormethyl-1,3,5-triazin

41,4 g (0,1 mol) des Kupferkomplexes aus Beispiel 3 werden in 300 ml DMF vorgelegt und 51,2 g (0,4 mol) Trifluoressigsäuremethylester bei 20°C innerhalb von 15 min zugetropft. Anschließend erwärmt man 1 Stunde auf 50°C und 5 Stunden auf 90°C. Die blaue Reaktionslösung wird eingeengt und der Rückstand mit 100 ml Wasser und 100 ml verdünnter Salzsäure aufgerührt. Nach dem Absaugen der Suspension, Waschen des Filterkuchens und Trocknen des Rückstands werden 23,4 g (0,12 mol, 60 %) des o.g. Triazins in Form eines farblosen Pulvers erhalten.

### Beispiel 5

### 2-Amino-4-ethoxy-6-trifluormethyl-1,3,5-triazin

8,4 g (0,1 mol)Cyanguanidin und 35,5 g (0,25 mol) Trifluoressigsäureethylester werden in 46 g Ethanol vorgelegt und eine Suspension von 8,5 g (0,125 mol) Natriumethylat in 39,8 g Ethanol innerhalb von 5 min zugegeben. Nach der Zugabe von 5,66 g (0,05 mol) Calciumchlorid wird die Reaktionsmischung 7 Std. auf Rückfluß erhitzt. Anschließend gibt man bei 20°C 0,5 ml konz. Salzsäure zu und entfernt das Ethanol. Es werden 100 g Wasser zugegeben und die Suspension abgesaugt. Der Rückstand wird mit 50 ml Wasser gewaschen und bei 50°C/20 mbar getrocknet. Man erhält 18,6 g (0,089 mol, 89 %) 2-Amino-4-ethoxy-6-trifluormethyl-1,3,5-triazin in Form eines farblosen Pulvers (HPLC: 99,8 Fl.-%, Fp: 124-125°C).

### Beispiel 6

### 2-Amino-4-difluormethyl-6-methoxy-1,3,5-triazin

Eine Lösung von 1,9 g (23 mmol) N-Cyanguanidin und 2,6 g (23 mmol) Calciumchlorid in 50 ml Methanol wird 90 min unter Rückfluß gerührt. Man kühlt das Reaktionsgemisch auf 20 bis 25°C ab und tropft rasch 5,0 g (45 mmol) Difluoressigsäuremethylester, dann langsam 4,1 g (23 mmol) Natriummethanolat-Lösung (30 gewichtsprozentig in Methanol) zu, worauf sich ein weißer Niederschlag abscheidet. Nach 2 Stunden Rühren unter Rückfluß entfernt man die flüchtigen Bestandteile im Wasserstrahlvakuum bei 40°C Badtemperatur, verteilt den Rückstand zwischen 100 ml Wasser und 100 ml Essigsäureethylester, trennt die organische Phase ab und trocknet diese über MgSO₄. Nach Entfernen des Lösungsmittels bei 40°C im Wasserstrahlvakuum verbleibt die Titelverbindung als leicht verunreinigtes Öl (1,1 g, 6,3 mmol; 28 % d. Th.), das bei Bedarf durch Verreiben mit einer Ether/Hexan-Mischung (v:v=1:3) kristallisiert werden kann.

¹H-NMR-Spektrum (400 MHz, CDCl₃, int. TMS, δ (ppm)): 7,44 br (1 H); 6,97 br (1 H); 6,27 t(²J_{H-F}=55 Hz, 1 H); 3,96 s (3 H). ¹³C-NMR-Spektrum (100 MHz, CDCl₃/CD₃S(O)CD₃, int. TMS, δ (ppm), protonenentkoppelt): 171,5 s (C-OCH₃); 169,8 t (C-CHF₂, ²J_{C-F}25 Hz); 168,8 s (C-NH₂); 111,3 t (CHF₂, ¹J_{C-F}243 Hz); 54,7 s (OCH₃).

## Patentansprüche

1. Verfahren zur Herstellung unsymmetrisch substituierter Triazine der allgemeinen Formel I 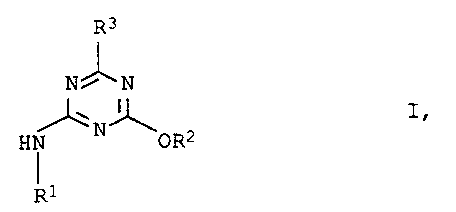 in der R¹ Wasserstoff, Methyl oder Ethyl, R² und R³ unabhängig voneinander einen Kohlenwasserstoffrest mit 1 bis 6 C-Atomen, der unter den Reaktionsbedingungen inerte Substituenten tragen kann, bedeuten, durch Umsetzung von Cyanguanidinen der Formel II 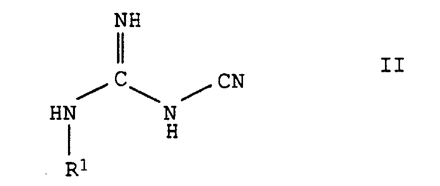 mit einem Carbonsäurederivat in Gegenwart eines Alkohols der Formel III
R²-OH III,
dadurch gekennzeichnet, daß man einen Carbonsäureester der Formel IV
R³-COOR⁴ IV,
in der R³ die obengenannte Bedeutung hat und R⁴ einen Kohlenwasserstoffrest mit 1 bis 6 C-Atomen darstellt, der unter den Reaktionsbedingungen inerte Substituenten tragen kann, in Gegenwart einer Base oder eines Carbonsäureamids, ausgewählt aus der Gruppe N,N-Di(C₁-C₄-alkyl)formamid, N,N-Di(C₁-C₄-alkyl)acetamid oder N-Methylpyrrolidon und in Gegenwart eines Salzes oder einer salzartigen Verbindung der Elemente Magnesium, Calcium, Aluminium, Zink, Kupfer, Eisen, Cobalt, Nickel oder Chrom umsetzt.

2. Verfahren nach Anspruch 1, dadurch gekennzeichnet, daß man als Base ein Alkoholat oder ein tertiäres Amin verwendet.

3. Verfahren nach Anspruch 1, dadurch gekennzeichnet, daß man als Base ein Alkali- oder Erdalkalialkoholat des Alkohols III verwendet.

4. Verfahren nach Anspruch 1, dadurch gekennzeichnet, daß man die o.g. Elemente in Form ihrer Halogenide, Nitrate, Sulfate, Alkoholate oder Acetate einsetzt.

5. Verfahren nach Anspruch 1, dadurch gekennzeichnet, daß man die Salze bzw. salzartigen Verbindungen gemäß Anspruch 1, in einer Menge von 0,001 bis 2 mol pro Mol Cyanguanidin II, verwendet.

6. Verfahren nach Anspruch 1, dadurch gekennzeichnet, daß man die Salze bzw. salzartigen Verbindungen von Schwermetallen gemäß Anspruch 1, in weniger als stöchiometrischen Mengen, bezogen auf das Cyanguanidin II, verwendet.

7. Verfahren nach Anspruch 1, dadurch gekennzeichnet, daß man Salze bzw. salzartige Verbindungen der Erdalkalimetalle Calcium oder Magnesium verwendet.

8. Verfahren nach Anspruch 6, dadurch gekennzeichnet, daß man die Erdalkalimetallverbindungen in einer Menge von 0,01 bis 2 mol pro Mol Cyanguanidin II verwendet.

9. Verfahren nach Anspruch 1, dadurch gekennzeichnet, daß man als Carbonsäureester den C₁-C₆-Alkylester einer perfluorierten oder perchlorierten C₁-C₃-Carbonsäure verwendet.

10. Verfahren nach Anspruch 1, dadurch gekennzeichnet, daß man Trifluoressigsäuremethylester in Methanol in Gegenwart eines Alkalimethanolats als Base umsetzt.

11. Verfahren nach Anspruch 1, dadurch gekennzeichnet, daß man Trifluoressigsäuremethylester in Gegenwart eines Alkalimethanolats und eines Calciumsalzes mit einem Cyanguanidin II umsetzt.

12. Verfahren nach Anspruch 1, dadurch gekennzeichnet, daß man Trifluoressigsäuremethylester in N,N-Dimethylformamid mit dem Cyanguanidin II in Gegenwart von Kupfersalzen, umsetzt.

13. Verfahren nach Anspruch 1, dadurch gekennzeichnet, daß man Trifluoressigsäuremethylester in Gegenwart eines Alkalimethanolats und eines Zinksalzes mit einem Cyanguanidin II umsetzt.

## Claims

1. A process for preparing asymmetrically substituted triazines of the general formula I 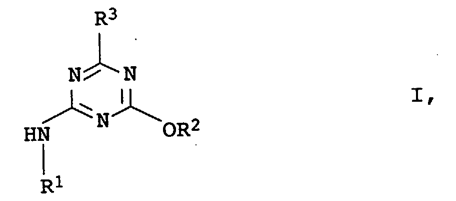 where R¹ is hydrogen, methyl or ethyl, R² and R³ independently of one another are a hydrocarbon radical having 1 to 6 C atoms, which can carry substituents which are inert under the reaction conditions, by reaction of a cyanoguanidine of the formula II 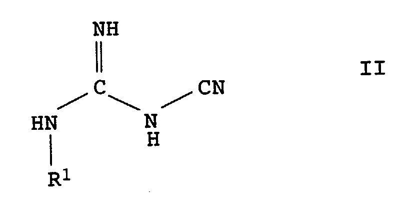 with a carboxylic acid derivative in the presence of an alcohol of the formula III
R²-OH III,
which comprises reacting a carboxylic acid ester of the formula IV
R³-COOR⁴ IV,
where R³ has the abovementioned meaning aid R⁴ is a hydrocarbon radical having 1 to 6 C atoms, which can carry substituents which are inert under the reaction conditions, in the presence of a base or of a carboxamide selected from the group consisting-of N,N-di(C₁-C₄-alkyl)formamide, N,N-di(C₁-C₄-alkyl)acetamide and N-methylpyrrolidone and in the presence of a salt or of a salt-like compound of the elements magnesium, calcium, aluminum, zinc, copper, iron, cobalt, nickel or chromium.

2. A process as claimed in claim 1, wherein the base used is an alkoxide or a tertiary amine.

3. A process as claimed in claim 1, wherein the base used is an alkali metal alkoxide or alkaline earth metal alkoxide of the alcohol III.

4. A process as claimed in claim 1, wherein the abovementioned elements are employed in the form of their halides, nitrates, sulfates, alkoxides or acetates.

5. A process as claimed in claim 1, wherein the salts or salt-like compounds as in claim 1 are used in an amount of from 0.001 to 2 mol per mole of cyanoguanidine II.

6. A process as claimed in claim 1, wherein the salts or salt-like compounds of heavy metals as in claim 1 are used in less than stoichiometric amounts, based on the cyanoguanidine II.

7. A process as claimed in claim 1, wherein salts or salt-like compounds of the alkaline earth metals calcium or magnesium are used.

8. A process as claimed in claim 6, wherein the alkaline earth metal compounds are used in an amount of from 0.01 to 2 mol per mole of cyanoguanidine II.

9. A process as claimed in claim 1, wherein the carboxylic acid esters used are the C₁-C₆-alkyl esters of a perfluorinated or perchlorinated C₁-C₃-carboxylic acid.

10. A process as claimed in claim 1, wherein methyl trifluoroacetate is reacted in methanol in the presence of an alkali methoxide as a base.

11. A process as claimed in claim 1, wherein methyl trifluoroacetate is reacted with a cyanoguanidine II in the presence of an alkali methoxide and of a calcium salt.

12. A process as claimed in claim 1, wherein methyl trifluoroacetate is reacted with the cyanoguanidine II in N,N-dimethylformamide in the presence of copper salts.

13. A process as claimed in claim 1, wherein methyl trifluoro-acetate is reacted with a cyanoguanidine II in the presence of an alkali methoxide and of a zinc salt.

## Revendications

1. Procédé pour la préparation de triazines substituées de façon asymétrique de formule générale I 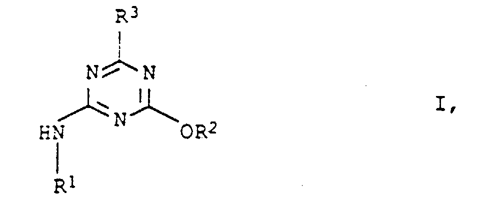 où R¹ représente l'hydrogène ou un groupe méthyle ou éthyle, R² et R³ représentent, indépendamment l'un de l'autre, un reste hydrocarboné ayant 1 à 6 atomes de carbone, qui peut porter des substituants inertes dans les conditions de la réaction, par réaction de cyanoguanidies de formule II 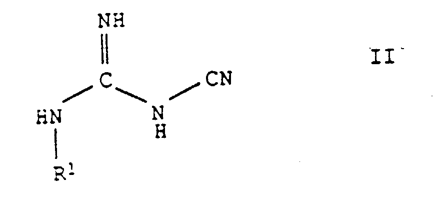 avec un dérivé d'acide carboxylique en présence d'un alcool de formule III
R²-OH III,
caractérisé par le fait qu'on fait réagir un ester d'acide carboxylique de formule IV
R³-COOR⁴ IV,
où R³ a la signification susdite et R⁴ représente un reste hydrocarboné ayant 1 à 6 atomes de carbone, qui peut porter des substituants inertes dans les conditions de la réaction, en présence d'une base ou d'un amide d'acide carboxylique, choisi parmi le N,N-di(alkyl en C₁-C₄)formamide, le N,N-di(alkyl en C₁-C₄)acétamide ou la N-méthylpyrrolidone et en présence d'un sel ou d'un composé de type sel des éléments magnésium, calcium, aluminium, zinc, cuivre, fer, cobalt, nickel ou chrome.

2. Procédé selon la revendication 1, caractérisé par le fait qu'on utilise comme base un alcoolate ou une amine tertiaire.

3. Procédé selon la revendication 1, caractérisé par le fait qu'on utilise comme base un alcoolate alcalin ou alcalino-terreux de l'alcool III.

4. Procédé selon la revendication 1, caractérisé par le fait qu'on emploie les éléments susmentionnés sous la forme de leurs halogénures, nitrates, sulfates, alcoolates ou acétates.

5. Procédé selon la revendication 1, caractérisé par le fait qu'on utilise les sels ou les composés de type sel selon la revendication 1, en une quantité de 0,001 à 2 mol par mol de cyanoguanidine II.

6. Procédé selon la revendication 1, caractérisé par le fait qu'on utilise les sels ou les composés de type sel de métaux lourds selon la revendication 1, en des quantités inférieures à la quantité stoechiométrique, par rapport à la cyanoguanidine II.

7. Procédé selon la revendication 1, caractérisé par le fait qu'on utilise des sels ou des composés de type sel des métaux alcalino-terreux calcium ou magnésium.

8. Procédé selon la revendication 6, caractérisé par le fait qu'on utilise les composés alcalino-terreux en une quantité de 0,01 à 2 mol par mol de cyanoguanidine II.

9. Procédé selon la revendication 1, caractérisé par le fait qu'on utilise comme ester d'acide carboxylique l'ester d'alkyle en C₁-C₆ d'un acide carboxylique en C₁-C₃ perfluoré ou perchloré.

10. Procédé selon la revendication 1, caractérisé par le fait qu'on fait réagir de l'ester méthylique d'acide trifluoroacétique dans du méthanol en présence d'un méthanolate alcalin en tant que base.

11. Procédé selon la revendication 1, caractérisé par le fait qu'on fait réagir de l'ester méthylique d'acide trifluoroacétique en présence d'un méthanolate alcalin et d'un sel de calcium avec une cyanoguanidine II.

12. Procédé selon la revendication 1, caractérisé par le fait qu'on fait réagir de l'ester méthylique d'acide trifluoroacétique en présence de N,N-diméthylformamide avec la cyanoguanidine II en présence de sels de cuivre.

13. Procédé selon la revendication 1, caractérisé par le fait qu'on fait réagir de l'ester méthylique d'acide trifluoroacétique en présence d'un méthanolate alcalin et d'un sel de zinc avec une cyanoguanidine II.
